# EUROPEAN PATENT APPLICATION

(11) **EP 4 775 153 A1**
(43) Date of publication of application: **15.07.2026**
(21) Application number: 23951386.4
(22) Date of filing: 28.12.2023
(51) Int. Cl.: A61B 10/04

(54) **MULTI-CHANNEL POLYP TRAP**

(30) Priority: 08.09.2023 CN 202322438469 U
(71) Applicant: SML Med-Tech Solutions Limited, Hong Kong (HK)
(72) Inventor: CHAU, King Yuen, Hong Kong (CN)
(74) Representative: Isern Patentes y Marcas S.L.
(86) International application number: PCT/CN2023/142776
(87) International publication number: WO 2025/050575

(57) **Abstract**

A multi-channel polyp trap, comprising a cup body (100); a collecting assembly (200), the collecting assembly (200) comprising a fixed base (210) and a plurality of trapping chambers (220), the fixed base (210) being arranged in the cup body (100), the plurality of trapping chambers (220) being detachably provided on the upper side of the fixed base (210), the plurality of trapping chambers (220) being evenly distributed in the circumferential direction of the fixed base (210), and the fixed base (210) being provided with a plurality of first snap-fit portions (211); and a cover body (300), the cover body (300) being rotatably provided on the cup body (100), the cover body (300) being detachably connected to the cup body (100), the cover body (300) being provided with a first guiding tube (310) and a second guiding tube (320), and the first guiding tube (310) being provided with a plurality of second snap-fit portions (311). The multi-channel polyp trap can ensure the second guiding tube (320) and a limiting recess to be correspondingly and accurately communicated and can provide tactile indications for medical staff so as to convey to the medical staff the information of the second guiding tube (320) being in-place, thereby reducing operation steps for medical staff, improving surgical efficiency, and further improving the quality of tissue specimens.

## Description

### Technical Field

The present application relates to the technical field of polyp traps, in particular to a multi-channel polyp trap.

### Background Art

Polyps refer to neoplasms growing on surfaces of human tissues. In modern medicine, neoplasms growing on human mucosal surfaces are generally collectively referred to as polyps, including hyperplastic tumors, inflammatory tumors, hamartomas, adenomas, and other tumors. For most polyps, endoscopic resection is the preferred treatment method. It is mainly achieved by completely resecting and removing the polyps from the mucosal surfaces using instruments such as a cutting instrument, an electrocautery knife, and biopsy forceps by inserting an endoscope into the body.

With the existing multi-channel polyp trap, a doctor needs to perform an additional step or operation to determine whether polyps can fall into the trap. Once negligence or omission occurs during a surgery, which results in incomplete polyp collection, an additional surgical or sampling process may be required to correct the error. This reduces the surgical efficiency, makes it difficult for the doctor to obtain accurate pathological evaluation results, and may even endanger the safety of a patient.

### Summary

The present application aims to solve at least one of the technical problems in the prior art. To this end, the present application provides a multi-channel polyp trap which can simplify an operation process and improve surgical efficiency, causing a doctor to accurately obtain a target tissue specimen, thereby improving quality of the tissue specimen.

A multi-channel polyp trap according to an embodiment of the present application, comprising a cup body; a collecting assembly, the collecting assembly including a fixed base and a plurality of trapping chambers, the fixed base being arranged in the cup body, the plurality of trapping chambers being detachably provided on an upper side of the fixed base, the plurality of trapping chambers being evenly distributed in a circumferential direction of the fixed base, a lower side of the fixed base and the cup body enclosing to form a cavity, a through hole being formed in a middle portion of the fixed base, and a plurality of first snap-fit portions being arranged on an inner wall of the through hole; and a cover body, the cover body being rotatably provided on the cup body, the cover body being detachably connected to the cup body, the cover body being provided with a first guiding tube and a second guiding tube, the first guiding tube and the cover body being coaxially provided, wherein one end of the first guiding tube away from the cover body may be connected to an endoscope; another end of the first guiding tube passes through the through hole and extends toward the cavity; a plurality of second snap-fit portions mating with the first snap-fit portions are arranged on an outer wall of the first guiding tube, and one end of the second guiding tube away from the cover body is connected to an air pump; when the cover body is rotated, the plurality of second snap-fit portions and the plurality of first snap-fit portions are alternately snapped, thereby causing the second guiding tube to be communicated with the plurality of trapping chambers in sequence.

The multi-channel polyp trap according to the embodiment of the present application at least has the following beneficial effects: During the resection operation, the endoscope connected to the first guiding tube enters a human body. After medical staff resects a polyp with an instrument, the polyp enters the second guiding tube along a pipeline under the action of the air pump, and finally falls into a limiting recess corresponding to the second guiding tube. After the previous polyp collection is completed, the medical staff rotates the cover body to cause four second snap-fit portions and four first snap-fit portions to be first separated from each other and then be in snap-fit with each other, to quickly fix the second guiding tube and the limiting recess, thereby ensuring that the second guiding tube and an idle limiting recess can be accurately communicated with each other correspondingly. The snap-fit between the second snap-fit portions and the first snap-fit portions provides a tactile indication to the medical staff and can convey to the medical staff information of the second guiding tube being in place, thereby reducing operation steps of the medical staff, improving surgical efficiency, and causing a doctor to accurately obtain a target tissue specimen, thereby improving quality of the tissue specimen.

According to some embodiments of the present application, a plurality of elastic members are arranged on the fixed base; the plurality of elastic members are located inside the cavity; the plurality of elastic members are evenly arranged around the first guiding tube; the plurality of first snap-fit portions are arranged on the plurality of elastic members in a one-to-one correspondence manner; the plurality of first snap-fit portions are respectively located on the sides of the plurality of elastic members close to the first guiding tube; the plurality of second snap-fit portions are arranged at one end of the first guiding tube located inside the cavity; and the plurality of second snap-fit portions are located on one side of the first guiding tube close to the elastic members.

According to some embodiments of the present application, the cover body is further provided with a plurality of third snap-fit portions; the plurality of third snap-fit portions are evenly arranged around the first guiding tube; the fixed base is provided with a cannula; the first guiding tube passes through the cannula and extends to an interior of the cavity; a plurality of fourth snap-fit portions respectively corresponding to the plurality of third snap-fit portions are arranged on one side of the cannula close to the cover body; the cover body is rotated to cause the second guiding tube to be alternately communicated with the plurality of trapping chambers in sequence; and the plurality of third snap-fit portions and the plurality of fourth snap-fit portions are correspondingly alternately snapped in sequence.

According to some embodiments of the present application, a plurality of bumps are arranged on one side of a side wall of the cover body close to a cup wall of the cup body; an annular groove is provided on one side of the cup wall of the cup body close to the cover body; a plurality of notches corresponding to the plurality of bumps are provided on an upper side of the annular groove; the plurality of bumps enter the annular groove through the plurality of notches; and the cover body is rotated to cause the plurality of bumps to move in a direction defined by the annular groove, thereby causing the cover body and the cup body to be rotatably connected.

According to some embodiments of the present application, a plurality of limiting blocks are arranged on the fixed base, and the plurality of limiting blocks are used for limiting positions of the trapping chambers on the fixed base.

According to some embodiments of the present application, a plurality of first through holes are provided at bottoms of the plurality of trapping chambers; a plurality of second through holes are provided on the fixed base; and gaps are reserved between the plurality of trapping chambers and the fixed base.

According to some embodiments of the present application, the cover body is provided with an identification portion; and the identification portion is used for indicating a communication state of the second guiding tube with the plurality of trapping chambers.

According to some embodiments of the present application, the second snap-fit portions are linear grooves arranged in an extension direction of the first guiding tube, and the first snap-fit portions are protrusions corresponding to the linear grooves.

According to some embodiments of the present application, the first guiding tube is provided with an alignment portion at a position away from the end connected to the endoscope; and the alignment portion is used for aligning the first guiding tube to pass through the fixed base and enter the cup body.

According to some embodiments of the present application, the first guiding tube is provided with a first transition portion; a cross-sectional area of the first transition portion gradually decreases in a direction away from the cover body; the second guiding tube is provided with a second transition portion; and a cross-sectional area of the second transition portion gradually decreases in the direction away from the cover body.

Additional aspects and advantages of the present application will be set forth in part in the following description, and in part will become apparent from the following description, or may be learned by practice of the present application.

### BRIEF DESCRIPTION OF THE DRAWINGS

The present application will be further described below in conjunction with the accompanying drawings and embodiments;
FIG. 1 is a schematic structural diagram of a multi-channel polyp trap according to an embodiment of the present application;
FIG. 2 is a schematic structural diagram of a collecting assembly in FIG. 1;
FIG. 3 is a schematic structural diagram of a cover body and the collecting assembly in FIG. 1;
FIG. 4 is a schematic structural diagram of the cover body in FIG. 1;
FIG. 5 is a cross-sectional view in FIG. 1; and
FIG. 6 is a schematic structural diagram of the collecting assembly and a cup body in FIG. 1.

### Reference numerals:

100: cup body; 110: annular groove; 111: notch;
200: collecting assembly; 210: fixed base; 211: first snap-fit portion; 212: elastic member; 213: cannula; 214: fourth snap-fit portion; 215: limiting block; 216: second through hole; 220: trapping chamber; 221: first through hole; 230: cavity;
300: cover body; 310: first guiding tube; 311: second snap-fit portion; 312: alignment portion; 313: first transition portion; 320: second guiding tube; 321: second transition portion; 330: bump; 340: third snap-fit portion; 350: identification portion.

### DETAILED DESCRIPTION

This section will describe the specific embodiments of the present application in detail. The preferred embodiments of the present application are illustrated in the accompanying drawings. The effect of the accompanying drawings is to supplement the textual description of this specification with graphs, so that a person can intuitively and visually understand each technical feature and the overall technical solution of the present application, but the accompanying drawings shall not be construed as limiting the protection scope of the present application.

In the description of the present application, it should be understood that regarding the description of orientations, orientations or positional relationships indicated by upper, lower, front, rear, left, right and the like are based on orientations or positional relationships shown in the accompanying drawings, and are used only for ease of the description of the present application and the simplification of description, rather than indicating or implying that the mentioned apparatus or element must have a particular orientation or be constructed and operated in a particular orientation. Therefore, these terms should not be construed as limiting the present application.

In the description of the present application, "a plurality of" means one or more. "Multiple" means two or more. "greater than", "less than", "exceeding", and the like are understood as excluding the original number; and "above", "below", "within", and the like are understood as including the original number. If there is a description of first and second, they are only for the purpose of distinguishing technical features, and cannot be understood as indicating or implying relative importance or implicitly indicating the quantity of the technical features or implicitly indicating the precedence relationship of the indicated technical features.

In the description of the present application, unless otherwise explicitly limited, words such as arrangement, mounting, and connection should be broadly understood. Those skilled in the art can properly determine the specific meanings of the above words in the present application in cooperation with specific content of technical solutions.

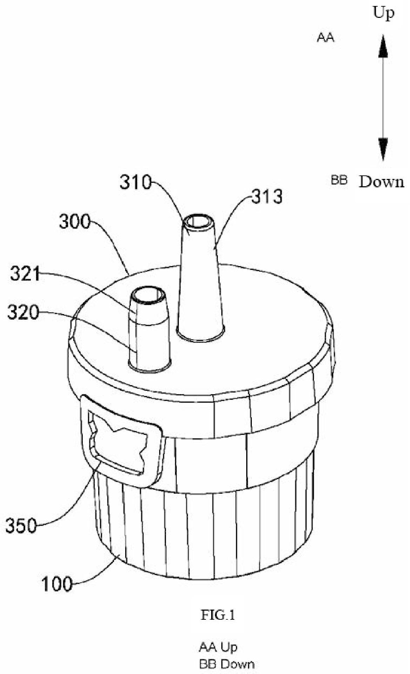

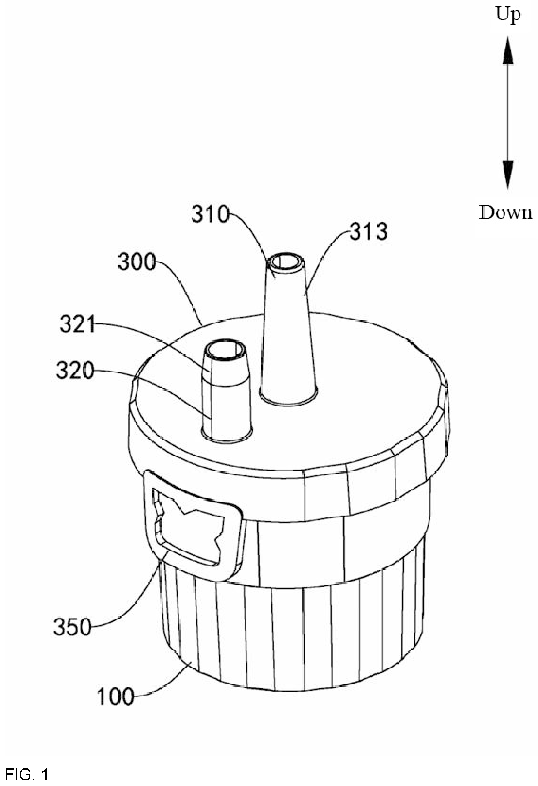

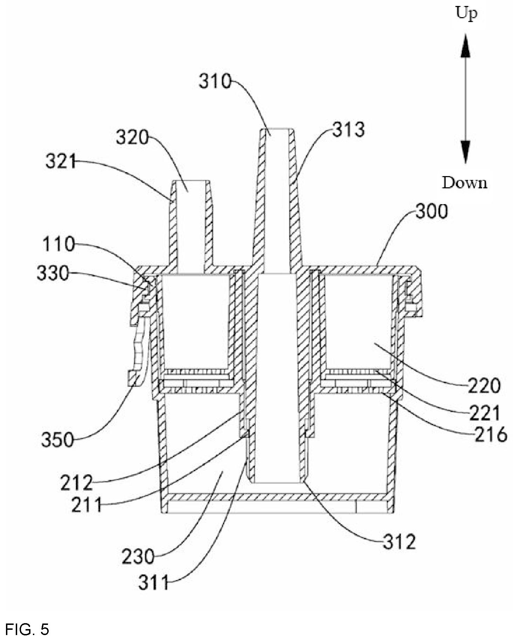

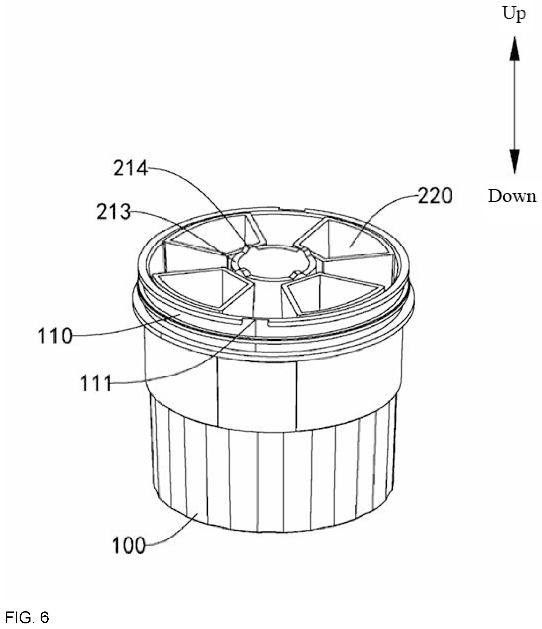

Referring to FIG. 1 to FIG. 6, a multi-channel polyp trap according to an embodiment of the present application is described.

As shown in FIG. 1 to FIG. 6, the multi-channel polyp trap according to the embodiment of the present application includes a cup body 100; a collecting assembly 200, the collecting assembly 200 comprising a fixed base 210 and a plurality of trapping chambers 220, the fixed base 210 being arranged in the cup body 100, the plurality of trapping chambers 220 being detachably provided on an upper side of the fixed base 210, the plurality of trapping chambers 220 being evenly distributed in a circumferential direction of the fixed base 210, a lower side of the fixed base 210 and the cup body 100 enclosing to form a cavity 230, a through hole being formed in a middle portion of the fixed base 210, and a plurality of first snap-fit portions 211 being arranged on an inner wall of the through hole; and a cover body 300, the cover body 300 being rotatably provided on the cup body 100, the cover body 300 being detachably connected to the cup body 100, the cover body 300 being provided with a first guiding tube 310 and a second guiding tube 320, the first guiding tube 310 and the cover body 300 being coaxially arranged, wherein one end of the first guiding tube 310 away from the cover body 300 may be connected to an endoscope, another end of the first guiding tube 310 passes through the through hole and extends toward the cavity 230, a plurality of second snap-fit portions 311 mating with the first snap-fit portions 211 are arranged on an outer wall of the first guiding tube 310, and one end of the second guiding tube 320 away from the cover body 300 is connected to an air pump; when the cover body 300 is rotated, the plurality of second snap-fit portions 311 and the plurality of first snap-fit portions 211 are alternately snapped, thereby causing the second guiding tube 320 to be communicated with the plurality of trapping chambers 220 in sequence.

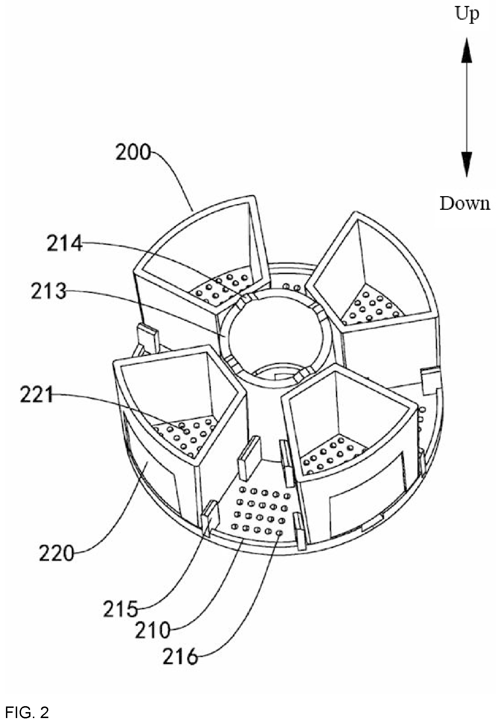

As shown in FIG. 1 and FIG. 2, any horizontal cross section of the cup body 100 is circular; the cup body 100 is provided with the fixed base 210; the fixed base 210 is a circular plate corresponding to an inner diameter of the cup body 100; an upper side of the fixed base 210 is detachably provided with four trapping chambers 220; the four trapping chambers 220 are evenly distributed around an axis of the fixed base 210; the lower side of the fixed base 210 and the cup body 100 enclose to form a cavity 230; and the fixed base 210 is provided with four first snap-fit portions 211. The cover body 300 is rotatably provided on the cup body 100, and the cover body 300 is detachably connected to the cup body 100; the cover body 300 is provided with the first guiding tube 310 and the second guiding tube 320; an upper end of the first guiding tube 310 is connected to the endoscope, and a lower end thereof passes through the fixed base 210 and extends toward the cavity 230 below; the first guiding tube 310 is provided with four second snap-fit portions 311; the four second snap-fit portions 311 can be respectively in snap-fit with the four first snap-fit portions 211 in a one-to-one correspondence manner; and the second guiding tube 320 extends upward, and an upper end thereof is connected to the air pump for receiving a fallen polyp. During the resection operation, the endoscope connected to the first guiding tube 310 enters a human body. After medical staff resects a polyp with an instrument, the polyp enters the second guiding tube 320 along a pipeline under the action of the air pump, and finally falls into a limiting recess corresponding to the second guiding tube 320. After the previous polyp collection is completed, the medical staff rotates the cover body 300 to cause four second snap-fit portions 311 and four first snap-fit portions 211 to be first separated from each other and then be in snap-fit with each other, to quickly fix the second guiding tube 320 and the limiting recess, thereby ensuring that the second guiding tube 320 and an idle limiting recess can be accurately communicated with each other correspondingly. The snap-fit between the second snap-fit portions 311 and the first snap-fit portions 211 provides a tactile indication to the medical staff and can convey to the medical staff information of the second guiding tube 320 being in place, thereby reducing operation steps of the medical staff, improving surgical efficiency, and causing a doctor to accurately obtain a target tissue specimen, thereby improving quality of the tissue specimen.

Specifically, the cup body 100, the cover body 300, the first guiding tube 310, the second guiding tube 320, and the trapping chambers 220 are all made of transparent materials, so as to ensure that medical staff can directly observe the collection of polyp tissues. It is conceivable that two, three, five, or more trapping chambers 220 can be provided; multiple trapping chambers 220 are evenly distributed in the circumferential direction of the fixed base 210, and correspondingly, at least one structure of the first snap-fit portions 211 and the second snap-fit portions 311 needs to be provided in a number corresponding to the number of trapping chambers 220, so as to ensure that at each time the second guiding tube 320 is correspondingly communicated with the trapping chambers 220 in sequence, the medical staff can obtain the information of being in place through touch or hearing. The multiple trapping chambers 220 can collect as many polyp tissues as possible without replacing the multi-channel polyp trap to simplify the operation. Certainly, the more trapping chambers 220 are provided, the less spaces of the trapping chambers 220 can accommodate polyps. However, it should be noted that a polyp is generally an abnormal tissue whose shape and position may pose challenges for effective specimen collection. Therefore, a properly sized trapping chamber 220 can provide better control and precision and enable a doctor to accurately obtain a target tissue specimen, thereby improving quality and reliability of sampling.

In some specific embodiments of the present application, a plurality of elastic members 212 are arranged on the fixed base 210; the plurality of elastic members 212 are located inside the cavity 230; the plurality of elastic members 212 are evenly arranged around the first guiding tube 310; the plurality of first snap-fit portions 211 are arranged on the plurality of elastic members 212 in a one-to-one correspondence manner; the plurality of first snap-fit portions 211 are respectively located on the sides of the plurality of elastic members 212 close to the first guiding tube 310; the plurality of second snap-fit portions 311 are arranged at one end of the first guiding tube 310 located inside the cavity; and the plurality of second snap-fit portions 311 are located on one side of the first guiding tube 310 close to the elastic members 212.

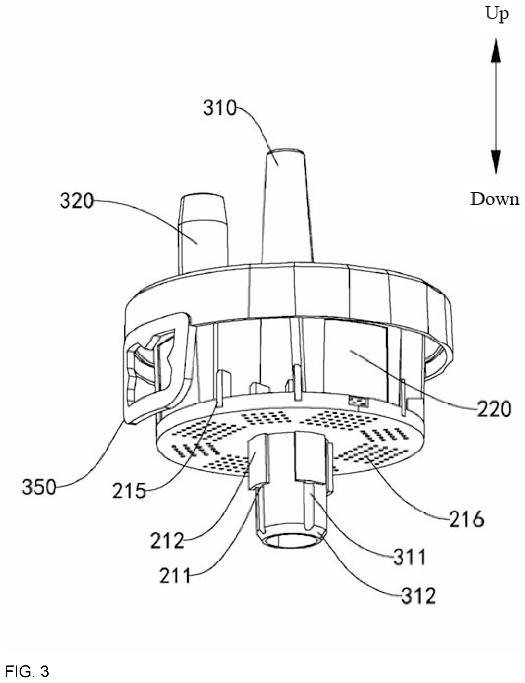

As shown in FIG. 3, four elastic members 212 are arranged on the lower side of the fixed base 210; the four elastic members 212 are arranged circumferentially around the first guiding tube 310, and the four elastic members 212 are respectively provided with the first snap-fit portions 211; and the first snap-fit portions 211 are located on the sides of the elastic members 212 close to the first guiding tube 310, correspondingly, four second snap-fit portions 311 are provided, the four second snap-fit portions 311 are arranged on one side, close to the elastic members 212, of one end of the first guiding tube 310 passing through the fixed base 210. The cover body 300 is rotated, and the four elastic members 212 deform in an axial direction away from the first guiding tube 310, so that the first guiding tube 310 can rotate relative to the fixed base 210 as the cover body 300 is rotated. In addition, the four second snap-fit portions 311 and the four first snap-fit portions 211 are alternately snapped respectively. Therefore, when the second guiding tube 320 reaches a set position and is correspondingly communicated with the trapping chambers 220, the four second snap-fit portions 311 and the four first snap-fit portions 211 are respectively snapped again one by one, so that medical staff can obtain information in real time during use.

In some specific embodiments of the present application, the cover body 300 is further provided with a plurality of third snap-fit portions 340; the plurality of third snap-fit portions 340 are evenly arranged around the first guiding tube 310; the fixed base 210 is provided with a cannula 213; the first guiding tube 310 passes through the cannula 213 and extends to an interior of the cavity 230; a plurality of fourth snap-fit portions 214 respectively corresponding to the plurality of third snap-fit portions 340 are arranged on one side of the cannula 213 close to the cover body 300; the cover body 300 is rotated to cause the second guiding tube 320 to be alternately communicated with the plurality of trapping chambers 220 in sequence; and the plurality of third snap-fit portions 340 and the plurality of fourth snap-fit portions 214 are correspondingly alternately snapped in sequence.

As shown in FIG. 2, FIG. 4, and FIG. 5, four third snap-fit portions 340 are arranged on a lower side surface of the cover body 300; the four third snap-fit portions 340 are evenly arranged circumferentially around the first guiding tube 310; the fixed base 210 is provided with the cannula 213; the first guiding tube 310 passes through the cannula 213 and enters the cavity 230; four fourth snap-fit portions 214 corresponding to the four third snap-fit portions 340 are arranged on an upper side of the cannula 213; and the four fourth snap-fit portions 214 and the four third snap-fit portions 340 can be alternately snapped. Therefore, in the process of rotating the cover body 300, the four third snap-fit portions 340 on the lower side of the cover body 300 can also rotate as the cover body 300 is rotated, at each time the second guiding tube 320 reaches a corresponding position and is correspondingly communicated with one of the four trapping chambers 220, the four third snap-fit portions 340 can be in snap-fit with the four fourth snap-fit portions 214 again, which in one aspect, strengthens the snap-fit reliability and the consistency of corresponding communication between the second guiding tube 320 and the trapping chambers 220, and in another aspect, further helps medical staff understand the state of the structure and reduces a possibility of accidents caused by misoperations.

In this specific embodiment, each third snap-fit portion 340 is a groove; specifically, the third snap-fit portion 340 is a groove with a trapezoidal vertical cross section; correspondingly, each fourth snap-fit portion 214 is a protrusion, and the fourth snap-fit portion 214 is a trapezoidal protrusion corresponding to the third snap-fit portion 340; a slope of the trapezoidal protrusion has a transition effect on the snap-fit of the structure, so as to ensure that the protrusion and the groove can be separated from each other by rotating the cover body 300 while the protrusion and the groove are snapped, thereby entering next snap-fit. Certainly, on the contrary, each third snap-fit portion 340 may be a protrusion, and each fourth snap-fit portion 214 may be a groove corresponding to the third snap-fit portion 340.

In some specific embodiments of the present application, a plurality of bumps 330 are arranged on one side of a side wall of the cover body 300 close to a cup wall of the cup body 100; an annular groove 110 is provided on one side of the cup wall of the cup body 100 close to the cover body 300; a plurality of notches 111 corresponding to the plurality of bumps 330 are provided on an upper side of the annular groove 110; the plurality of bumps 330 enter the annular groove 110 through the plurality of notches 111; the cover body 300 is rotated to cause the plurality of bumps 330 to move in a direction defined by the annular groove 110, thereby causing the cover body 300 and the cup body 100 to be rotatably connected.

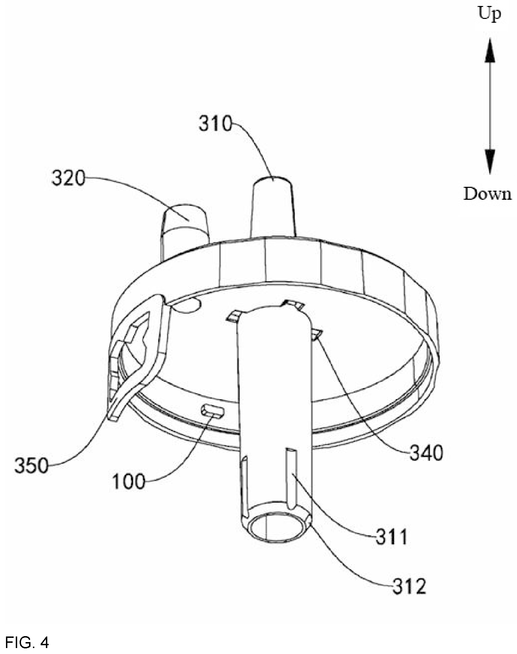

As shown in FIG. 4 to FIG. 6, two bumps 330 are arranged on the side of an annular side wall of the cover body 300 close to the cup wall of the cup body 100; the annular groove 110 is provided on the side of the cup wall of the cup body 100 close to a side wall of the cover body 300; and two notches 111 corresponding to the two bumps 330 are provided on the upper side of the annular groove 110. In this specific embodiment, the two bumps 330 are arranged oppositely, correspondingly, the two notches 111 are arranged oppositely; the cover body 300 is closed downward from a top of the cup body 100; firstly, the two bumps 330 pass through the two notches 111 corresponding to the two bumps and enter the annular groove 110; the cover body 300 is then rotated to cause the two bumps 330 to move within the annular groove 110 along a channel defined by the annular groove, thereby achieving a detachable rotatable connection between the cover body 300 and the cup body 100. Certainly, one, three, four, or more bumps 330 can also be provided, and one, three, four, or more notches 111 also need to be provided correspondingly, so that the plurality of bumps 330 can pass through the plurality of notches 111 and enter the annular groove 110.

It is conceivable that, on the contrary, the plurality of bumps 330 can be arranged on the side of the cup wall of the cup body 100 close to the cover body 300; the annular groove 110 can be provided on the side of the side wall of the cover body 300 close to the cup wall of the cup body 100; the plurality of notches 111 corresponding to the plurality of bumps 330 are provided on the upper side of the annular groove 110; the plurality of bumps 330 enter the annular groove 110 through the plurality of notches 111; and rotating the cover body 300 can also cause the plurality of bumps 330 to move in the direction defined by the annular groove 110 and cause the cover body 300 and the cup body 100 to be rotatably connected.

In some specific embodiments of the present application, a plurality of limiting blocks 215 are arranged on the fixed base 210, and the plurality of limiting blocks 215 are used for limiting positions of the trapping chambers 220 on the fixed base 210.

In some specific embodiments of the present application, a plurality of first through holes 221 are provided at bottoms of the plurality of trapping chambers 220; a plurality of second through holes 216 are provided on the fixed base 210; and gaps are reserved between the plurality of trapping chambers 220 and the fixed base 210.

As shown in FIG. 2 and FIG. 3, a plurality of limiting blocks 215 are arranged on the fixed base 210. In this specific embodiment, each trapping chamber 220 corresponds to four limiting blocks 215; and each trapping chamber 220 is inserted in a position defined by the four limiting blocks 215. The plurality of first through holes 221 are provided at the bottoms of the trapping chambers 220, and the plurality of second through holes 216 are also provided on the fixed base 210. Specifically, an edge of the fixed base 210 has an annular ridge, the trapping chambers 220 are erected on the annular ridge, and positions of the trapping chambers 220 are defined by the plurality of limiting blocks 215 to form the gaps between the trapping chambers 220 and the fixed base 210. Therefore, when the polyp falls into the trapping chambers 220, substances except polyp tissues can be filtered into the cavity 230 by dual filtering of the first through holes 221 and the second through holes 216, so as to ensure that medical staff can effectively collect and store the polyp tissues and prevent loss or contamination of the polyp tissues during collection.

In some specific embodiments of the present application, the cover body 300 is provided with an identification portion 350, and the identification portion 350 is used for indicating a communication state of the second guiding tube 320 and the plurality of trapping chambers 220.

As shown in FIG. 3, the identification portion 350 is arranged on a side wall of the cover body 300, and when the cover body 300 is rotated to cause the second guiding tube 320 to correspond to a trapping chamber 220, the identification portion 350 points to the trapping chamber 220. In this specific embodiment, the identification portion 350 is of a frame-shaped structure, and triangular protrusions are arranged on an upper side and left and right sides of the frame-shaped structure; the triangular protrusions all point to the trapping chamber 220 exposed out of a center of the frame-shaped structure, and medical staff is provided with a visual confirmed structure to determine a corresponding state of the second guiding tube 320 and the trapping chamber 220. This further expands the use value of the multi-channel polyp trap.

In some specific embodiments of the present application, the second snap-fit portions 311 are linear grooves arranged in an extension direction of the first guiding tube 310.

As shown in FIG. 3, the second snap-fit portions 311 are the linear grooves provided in the extension direction of the first guiding tube 310, and correspondingly, the first snap-fit portions 211 are protrusions capable of moving along the linear grooves. In the process of relative rotation of the cover body 300 and the cup body 100, the cover body 300 and the cup body 100 undergo relative movement in an up-down direction. Therefore, designing the grooves to be in a shape of a long strip can enlarge their contact areas with the protrusions, so as to provide more contact points and increase the stability and tightness of connection. Furthermore, in the process that the first guiding tube 310 is inserted into the cannula 213, the linear grooves can guide the protrusions to be in snap-fit, causing the protrusions to more easily slide into the linear grooves, so as to improve the accuracy of positioning, reduce deviations and errors in the assembly process, and simplify the mounting and removal processes of an assembly.

In some specific embodiments of the present application, the first guiding tube 310 is provided with an alignment portion 312 away from the end connected to the endoscope, and the alignment portion 312 is used for aligning the first guiding tube 310 to pass through the fixed base 210 and enter the cup body 100.

As shown in FIG. 3 and FIG. 4, the alignment portion 312 is arranged at a lower end of the first guiding tube 310; a cross-sectional area of the alignment portion 312 gradually increases from bottom to top, and the alignment portion 312 can align a direction of the first guiding tube 310 when the first guiding tube 310 is inserted into the cannula 213 of the fixed base 210.

In some specific embodiments of the present application, the first guiding tube 310 is provided with a first transition portion 313; a cross-sectional area ofthe first transition portion 313 gradually decreases in a direction away from the cover body 300; the second guiding tube 320 is provided with a second transition portion 321; and a cross-sectional area of the second transition portion 321 gradually decreases in the direction away from the cover body 300.

As shown in FIG. 1, FIG. 3, and FIG. 4, the first transition portion 313 is arranged at an upper end of the first guiding tube 310, the cross-sectional area of the first transition portion 313 gradually decreases from bottom to top, the second transition portion 321 is arranged at an upper end of the second guiding tube 320; the cross-sectional area of the second transition portion 321 gradually decreases from bottom to top; when the first guiding tube 310 and the second guiding tube 320 need to be connected to equipment through pipelines, by providing the first transition portion 313 and the second transition portion 321, a tight connection can be ensured between connection ports, thereby lowering a risk of air or liquid leakage. Furthermore, when the second guiding tube 320 is connected to the air pump, the second transition portion 321 can provide a larger contact area to withstand a pressure or tension to lower a risk of loosening or separation of the connection ports. Meanwhile, the arrangement of the first transition portion 313 and the second transition portion 321 can also simplify the mounting process, so that the two connection ports are more easily aligned during mounting, and difficulties and errors during mounting are reduced. This helps improve the working efficiency and reduce problems caused by the mounting errors.

The embodiments of the present application have been described in detail above with reference to the accompanying drawings. However, the present application is not limited to the foregoing embodiments. Various changes can be made within the knowledge scope of a person of ordinary skill in the art without departing from the purpose of the present application.

## Claims

1. A multi-channel polyp trap, **characterized in that** it comprises:
a cup body (100);
a collecting assembly (200), the collecting assembly (200) comprising a fixed base (210) and a plurality of trapping chambers (220), the fixed base (210) being arranged in the cup body (100), the plurality of trapping chambers (220) being detachably provided on an upper side of the fixed base (210), the plurality of trapping chambers (220) being evenly distributed in a circumferential direction of the fixed base (210), a lower side of the fixed base (210) and the cup body (100) enclosing to form a cavity (230), a through hole being formed in a middle portion of the fixed base (210), and a plurality of first snap-fit portions (211) being arranged on an inner wall of the through hole; and
a cover body (300), the cover body (300) being rotatably provided on the cup body (100), the cover body (300) being detachably connected to the cup body (100), the cover body (300) being provided with a first guiding tube (310) and a second guiding tube (320), the first guiding tube (310) and the cover body (300) being coaxially arranged, wherein one end of the first guiding tube (310) away from the cover body (300) may be connected to an endoscope, another end of the first guiding tube (310) passes through the through hole and extends toward the cavity (230), a plurality of second snap-fit portions (311) mating with the first snap-fit portions (211) are arranged on an outer wall of the first guiding tube (310), and one end of the second guiding tube (320) away from the cover body (300) is connected to an air pump;
when the cover body (300) is rotated, the plurality of second snap-fit portions (311) and the plurality of first snap-fit portions (211) are alternately snapped, thereby causing the second guiding tube (320) to be communicated with the plurality of trapping chambers (220) in sequence.

2. The multi-channel polyp trap according to claim 1, **characterized in that** a plurality of elastic members (212) are arranged on the fixed base (210); the plurality of elastic members (212) are located inside the cavity (230); the plurality of elastic members (212) are evenly arranged around the first guiding tube (310); the plurality of first snap-fit portions (211) are arranged on the plurality of elastic members (212) in a one-to-one correspondence manner; the plurality of first snap-fit portions (211) are respectively located on the sides of the plurality of elastic members (212) close to the first guiding tube (310); the plurality of second snap-fit portions (311) are arranged at one end of the first guiding tube (310) located at the cavity; and the plurality of second snap-fit portions (311) are located on one side of the first guiding tube (310) close to the elastic members (212).

3. The multi-channel polyp trap according to claim 1, **characterized in that** the cover body (300) is further provided with a plurality of third snap-fit portions (340); the plurality of third snap-fit portions (340) are evenly arranged around the first guiding tube (310); the fixed base (210) is provided with a cannula (213); the first guiding tube (310) passes through the cannula (213) and extends to an interior of the cavity (230); a plurality of fourth snap-fit portions (214) respectively corresponding to the plurality of third snap-fit portions (340) are arranged on one side of the cannula (213) close to the cover body (300); the cover body (300) is rotated to cause the second guiding tube (320) to be alternately communicated with the plurality of trapping chambers (220) in sequence; and the plurality of third snap-fit portions (340) and the plurality of fourth snap-fit portions (214) are correspondingly alternately snapped in sequence.

4. The multi-channel polyp trap according to claim 1, **characterized in that** a plurality of bumps (330) are arranged on one side of a side wall of the cover body (300) close to a cup wall of the cup body (100); an annular groove (110) is provided on one side of the cup wall of the cup body (100) close to the cover body (300); a plurality of notches (111) corresponding to the plurality of bumps (330) are provided on an upper side of the annular groove (110); the plurality of bumps (330) enter the annular groove (110) through the plurality of notches (111); and the cover body (300) is rotated to cause the plurality of bumps (330) to move in a direction defined by the annular groove (110), thereby causing the cover body (300) and the cup body (100) to be rotatably connected.

5. The multi-channel polyp trap according to claim 1, **characterized in that** a plurality of limiting blocks (215) are arranged on the fixed base (210), and the plurality of limiting blocks (215) are used for limiting positions of the trapping chambers (220) on the fixed base (210).

6. The multi-channel polyp trap according to claim 1, **characterized in that** a plurality of first through holes (221) are provided at bottoms of the plurality of trapping chambers (220); a plurality of second through holes (216) are provided on the fixed base (210); and gaps are reserved between the plurality of trapping chambers (220) and the fixed base (210).

7. The multi-channel polyp trap according to claim 1, **characterized in that** the cover body (300) is provided with an identification portion (350); and the identification portion (350) is used for indicating a communication state of the second guiding tube (320) with the plurality of trapping chambers (220).

8. The multi-channel polyp trap according to claim 1, **characterized in that** the second snap-fit portions (311) are linear grooves arranged in an extension direction of the first guiding tube (310), and the first snap-fit portions (211) are protrusions corresponding to the linear grooves.

9. The multi-channel polyp trap according to claim 1, **characterized in that** the first guiding tube (310) is provided with an alignment portion (312) at a position away from the end connected to the endoscope; and the alignment portion (312) is used for aligning the first guiding tube (310) to pass through the fixed base (210) and enter the cup body (100).

10. The multi-channel polyp trap according to claim 1, **characterized in that** the first guiding tube (310) is provided with a first transition portion (313); a cross-sectional area of the first transition portion (313) gradually decreases in a direction away from the cover body (300); the second guiding tube (320) is provided with a second transition portion (321); and a cross-sectional area of the second transition portion (321) gradually decreases in the direction away from the cover body (300).
